(19) Europäisches Patentamt
European Patent Office
Office européen des brevets

(11) Veröffentlichungsnummer: **0 150 034**
**B1**

(12) # EUROPÄISCHE PATENTSCHRIFT

(45) Veröffentlichungstag der Patentschrift: **02.08.89**

(21) Anmeldenummer: **85100349.1**

(22) Anmeldetag: **15.01.85**

(51) Int. Cl.⁴: **C 07 D 495/04**, C 07 D 333/38, C 09 B 29/06 // (C07D495/04, 333:00, 209:00), (C07D495/04, 333:00, 237:00)

(54) **2-Aminothiophenderivate.**

(30) Priorität: **21.01.84 DE 3402026**

(43) Veröffentlichungstag der Anmeldung: **31.07.85 Patentblatt 85/31**

(45) Bekanntmachung des Hinweises auf die Patenterteilung: **02.08.89 Patentblatt 89/31**

(84) Benannte Vertragsstaaten: **CH DE FR GB IT LI**

(56) Entgegenhaltungen:
**DE-A-2 304 201**
**DE-A-2 509 922**
**DE-A-2 513 337**
**DE-A-2 553 621**
**GB-A-1 389 266**

**Chemische Berichte, volume 98, page 3571 (1965) and volume 99 at page 94 (1966)**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Eilingsfeld, Heinz, Dr., Pierstrasse 9a, D-6710 Frankenthal (DE)**
Erfinder: **Etzbach, Karl- Heinz, Dr., Bensheimer Ring 9a, D-6710 Frankenthal (DE)**

**EP 0 150 034 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft Verbindungen der allgemeinen Formel I

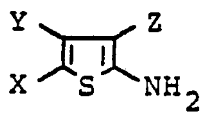 I,

in der

X und Y unabhängig voneinander Carboxyl, $C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxy)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, gegebenenfalls durch Chlor, Brom, Nitro oder Cyan substituiertes Phenoxycarbonyl, $C_1$- bis $C_4$-Alkylphenoxycarbonyl, Dimethylphenoxycarbonyl, Trimethylphenoxycarbonyl, Carbamoyl, $C_1$- bis $C_{12}$-Alkylcarbamoyl, Cyclohexylcarbamoyl, Allylcarbamoyl, 2-($C_1$- bis $C_4$-Alkoxy)-ethylcarbamoyl, 3-($C_1$- bis $C_4$-Alkoxy)-propylcarbamoyl, 2-Phenoxyethylcarbamoyl, 3-Phenoxypropylcarbamoyl, Phenylmethylcarbamoyl, 2-Phenylethylcarbamoyl, Phenylcarbamoyl, gegebenenfalls durch Chlor, Brom, Nitro oder Cyan substituiertes Phenylcarbamoyl, $C_1$- bis $C_{12}$-Alkylphenylcarbamoyl, Dimethylphenylcarbamoyl, Trimethylphenylcarbamoyl, $C_1$- bis $C_4$-Dialkylcarbamoyl, Methyl-cyclohexylcarbamoyl, Dicyclohexylcarbamoyl oder Reste der Formel

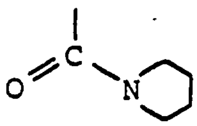 , 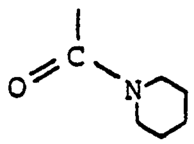 oder 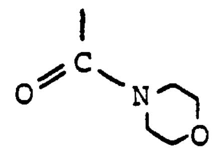 ,

X und Y zusammen ein Rest der Formel

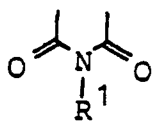 oder 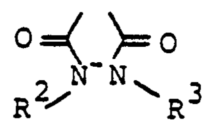 und

Z Cyan, Carboxyl, $C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxy)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, gegebenenfalls durch Chlor, Brom, Nitro oder Cyan substituiertes Phenoxycarbonyl, $C_1$- bis $C_4$-Alkylphenoxycarbonyl, Dimethylphenoxycarbonyl, Trimethylphenoxycarbonyl, Carbamoyl, $C_1$- bis $C_{12}$-Alkylcarbamoyl, Cyclohexylcarbamoyl, Allylcarbamoyl, 2-($C_1$- bis $C_4$-Alkoxy)-ethylcarbamoyl, 3-($C_1$- bis $C_4$-Alkoxy)-propylcarbamoyl, 2-Phenoxyethylcarbamoyl, 3-Phenoxypropylcarbamoyl, Phenylmethylcarbamoyl, 2-Phenylethylcarbamoyl, Phenylcarbamoyl, gegebenenfalls durch Chlor, Brom, Nitro oder Cyan substituiertes Phenylcarbamoyl, $C_1$- bis $C_{12}$-Alkylphenylcarbamoyl, Dimethylphenylcarbamoyl, Trimethylphenylcarbamoyl, $C_1$- bis $C_4$-Dialkylcarbamoyl, Methyl-cyclohexylcarbamoyl, Dicyclohexylcarbamoyl oder Reste der Formel

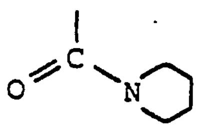 , 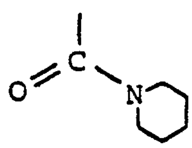 oder 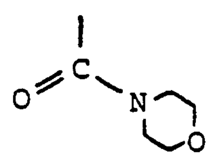 ,

sind, wobei

$R^1$ Wasserstoff, $C_1$- bis $C_{12}$-Alkyl, Cyclohexyl, Allyl, 2-($C_1$- bis $C_4$-Alkoxy)-ethyl, 3-($C_1$- bis $C_4$-Alkoxy)-propyl, 2-Phenoxyethyl, 3-Phenoxypropyl, Phenylmethyl, Phenylethyl oder gegebenenfalls durch Chlor, Brom, Cyan oder Nitro substituiertes Phenyl, $C_1$- bis $C_{12}$-Alkylphenyl, Dimethylphenyl oder Trimethylphenyl und

$R^2$ und $R^3$ Wasserstoff oder $C_1$- bis $C_{12}$-Alkyl oder einer der Reste $R^2$ und $R^3$ auch gegebenenfalls durch Chlor, Brom, Cyan oder Nitro substituiertes Phenyl sind.

Wenn die Carbamoylverbindungen durch Öffnung des Ringes

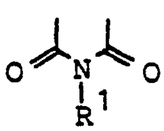

hergestellt werden, bleibt die Gruppe der Formel $-CONHR^1$ erhalten, die zweite Carbamoylgruppe, die entweder X oder Y entspricht, wird mit einem zur Ringspaltung verwendeten Amin gebildet.

Gleiches gilt entsprechend für die Ester und Säureverbindungen.

Zur Herstellung der Verbindungen der Formel I kann man Verbindungen der Formel II

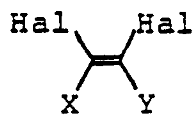 II

mit Verbindungen der Formel

$Z-CH_2-CN$

umsetzen und die Zwischenprodukte mit Schwefelwasserstoff in die Verbindungen der Formel I überführen.

Verbindungen der Formel II sind z. B. in J. Prakt. Chem. 321, 787 (1979) von M. Augustin, G. Fischer, B. Schneider und M. Köhler, in J. Am. Chem. Soc. 75, 4338 (1953) von N. R. Eldred und D. M. Young und in Zh. Obshch. Khim. 24, 1216 (1954) von Yu. A. Baskakov und N. N. Melnikov beschrieben.

In der GB-A-1 389 266 wird 2-Amino-3,4,5-tris(ethoxycarbonyl)thiophen als Komponente genannt, jedoch wird keine Lehre gegeben, wie diese Verbindung herzustellen ist. Andere dort genannte 2-Aminothiophene führen ebenfalls bei ihrer Verwendung als Diazokomponenten zu Azofarbstoffen.

Repräsentative Einzelheiten der Herstellung können den Beispielen entnommen werden, in denen sich Angaben über Teile und Prozente, sofern nicht anders vermerkt, auf das Gewicht beziehen.

Die Verbindungen der Formel I sind diazotierbar und eignen sich daher z. B. als Diazokomponenten.

Von besonderer Bedeutung sind Verbindungen der Formel I a

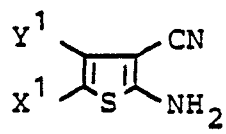 I a,

in der

$X^1$ und $Y^1$ unabhängig voneinander Carboxyl, $C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxy)-propyloxycarbonyl, 2-phenoxyethoxycarbonyl, 3-Phenoxypropyloxycarbonyl, Phenylmethoxycarbonyl, 2-Phenylethoxycarbonyl, Phenoxycarbonyl, gegebenenfalls durch Chlor, Brom, Nitro oder Cyan substituiertes Phenoxycarbonyl, $C_1$- bis $C_4$-Alkylphenoxycarbonyl, Dimethylphenoxycarbonyl oder Trimethylphenoxycarbonyl oder

$X^1$ und $Y^1$ zusammen ein Rest der Formel

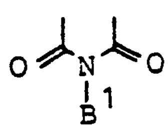

sind.

Bevorzugte Carbonestergruppen sind z. B.:

$C_1$- bis $C_{12}$-Alkoxycarbonyl, Cyclohexylcarbonyl, Phenylmethoxycarbonyl, Phenylethoxycarbonyl oder Phenoxycarbonyl.

Bevorzugte Reste $B^1$ sind z. B.:

Wasserstoff, $C_1$- bis $C_{12}$-Alkyl, Cyclohexyl, Phenylmethyl, Phenylethyl, Phenyl, $C_1$- bis $C_{12}$-Alkylphenyl, Dimethylphenyl, Trimethylphenyl oder durch Chlor, Brom, Cyan oder Nitro substituiertes Phenyl.

Weiter von besonderer Bedeutung sind Verbindungen der Formel I a, bei denen $X^1$ und $Y^1$ unabhängig voneinander $CONHB^1$ sind wobei $B^1$ die angegebene Bedeutung hat.

Weiter sind Verbindungen der Formel

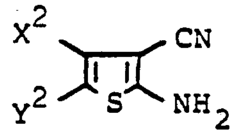

ebenfalls wertvoll, in der einer der Reste $X^2$ und $Y^2$ $CONHB^1$ und der andere Di-($C_1$- bis $C_{12}$-Alkyl)-carbamoyl, Carboxyl, $C_1$- bis $C_{12}$-Alkoxycarbonyl, Phenoxycarbonyl oder ein Rest der Formel

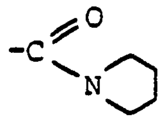, 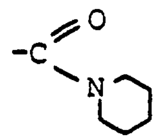 oder 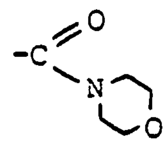

sind und

$B^1$ die angegebene Bedeutung hat.

Weiterhin sind Verbindungen der Formel

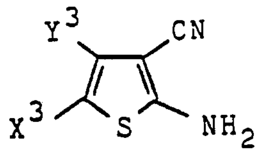

wertvoll in der

$X^3$ $C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxy)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, gegebenenfalls durch Chlor, Brom, Nitro oder Cyan substituiertes Phenoxycarbonyl, $C_1$- bis $C_4$-Alkylphenoxycarbonyl, Dimethylphenoxycarbonyl oder Trimethylphenoxycarbonyl und

$Y^3$ Carboxyl, Cyan, Carbamoyl, $C_1$- bis $C_{12}$-Alkylcarbamoyl, Cyclohexylcarbamoyl, Allylcarbamoyl, 2-($C_1$- bis $C_4$-Alkoxy)-ethylcarbamoyl, 3-($C_1$- bis $C_4$-Alkoxy)-propylcarbamoyl, 2-Phenoxyethylcarbamoyl, 3-Phenoxypropylcarbamoyl, Phenylmethylcarbamoyl, 2-Phenylethylcarbamoyl, Phenylcarbamoyl, gegebenenfalls durch Chlor, Brom, Nitro oder Cyan substituiertes Phenylcarbamoyl, $C_1$- bis $C_{12}$-Alkylphenylcarbamoyl, Dimethylphenylcarbamoyl, Trimethylphenylcarbamoyl, $C_1$- bis $C_4$-Dialkylcarbamoyl, Methyl-cyclohexylcarbamoyl, Dicyclohexylcarbamoyl oder Reste der Formel

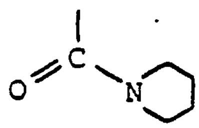, 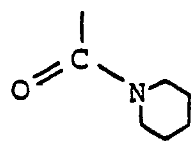 oder 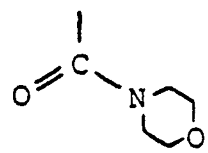,

$C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxy)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, gegebenenfalls durch Chlor, Brom, Nitro oder Cyan substituiertes Phenoxycarbonyl, $C_1$- bis $C_4$-Alkylphenoxycarbonyl, Dimethylphenoxycarbonyl oder Trimethylphenoxycarbonyl sind.

**Beispiel 1**

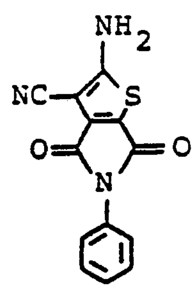

Zu einer Mischung aus 484 Teilen Dichlor-N-phenylmaleinimid, 132 Teilen Malonsäuredinitril und 1700 Teilen Methanol werden bei 0°C 720 Teile einer 30-%-igen methanolischen Natriummethylatlösung gegeben. Die Mischung wird noch 1 h bei 0°C gerührt, dann gibt man 240 Teile Eisessig zu und leitet anschließend bei 0°C 102 Teile Schwefelwasserstoff in die Lösung ein. Danach wird das Kühlbad entfernt und das Reaktionsgemisch, das sich zunächst auf 30 - 40°C erwärmt, über Nacht gerührt. Der entstandene Niederschlag wird abgesaugt, mit wenig Methanol, dann mit Eisessig und anschließend mit Wasser gewaschen und getrocknet. Man erhält 308 Teile schwefelhaltiges Rohprodukt, welches zur Reinigung in 1000 Teilen Dimethylformamid gelöst wird; zu dieser Lösung gibt man dann 40 Teile Tierkohle, rührt 8,5 h bei Raumtemperatur und filtriert die Mischung. Das Filtrat wird in 3500 Teile Wasser gegeben, der gelbe Niederschlag abgesaugt, mit Wasser gewaschen und getrocknet. Man erhält 258 Teile (48 % der Theorie) 2-Amino-3-cyan-thiophen-(4,5)-N-phenyldicarboximid.

Schmp.: 295°C (Eisessig), IR (KBr): 3380, 3311, 3204 ($NH_2$), 2225 (C≡N), 1767, 1708, 1654, 1644 $cm^{-1}$ (C=O)

$C_{13}H_7N_3O_2S$ (269,28)

| | | | | | |
|---|---|---|---|---|---|
| Ber.: | C 57,99 | H 2,62 | N 15,60 | O 11,88 | S 11,91 |
| Gef.: | C 57,9 | H 2,8 | N 14,4 | O 12,3 | S 11,7 |

Die Verbindungen der Beispiele 2 - 6 werden analog dem für Beispiel 1 beschriebenen Verfahren hergestellt.

**Beispiel 2**

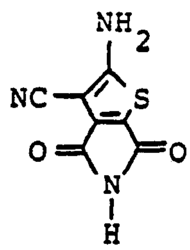

Schmp.: $>$ 300 °C (Eisessig)

**Beispiel 3**

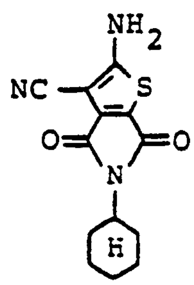

Schmp.: 244 °C (Eisessig)

**Beispiel 4**

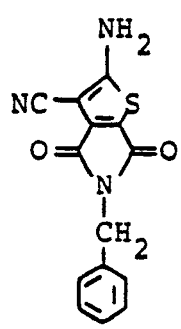

Schmp.: 231 °C (Eisessig)

**Beispiel 5**

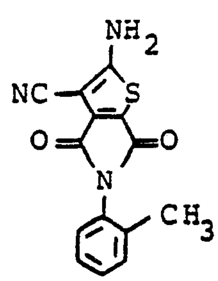

Schmp.: 237 °C (Eisessig)

**Beispiel 6**

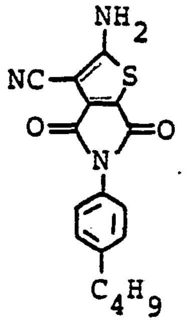

Schmp.: 220 °C (Eisessig)

**Beispiel 7**

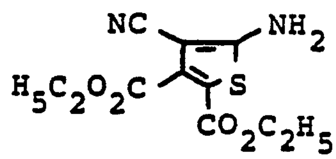

Zu einer Lösung von 3,2 Teilen Malonsäuredinitril in 40 Teilen Tetrahydrofuran gibt man portionsweise unter Eiskühlung 2,4 Teile Natriumhydrid und rührt anschließend noch 0,5 h bei 0°C. Nach Zugabe von 12,1 Teilem Dichlormaleinsäurediethylester bei 0°C rührt man das Gemisch noch 1 h bei Raumtemperatur, gibt dann 4 Teile Pyridin zu und leitet anschließend 3 Teile Schwefelwasserstoff in die Mischung ein. Nach vierständigem Rühren bei Raumtemperatur gibt man 300 Teile Wasser zu, extrahiert die Mischung zweimal mit Methylenchlorid, wäscht die vereinigten organischen Phasen mit Wasser, dann mit einer gesättigten wäßrigen Natriumhydrogencarbonatlösung und dann nochmals mit Wasser und trocknet sie mit Natriumsulfat. Nach dem Abfiltrieren des Trockenmittels und dem Abziehen des Methylenchlorids im Vakuum erhält man 9,4 Teile (70 % der Theorie) 2-Amino-3-cyan-thiophen-4,5-dicarbonsäurediethylester als rotes, allmählich kristallisierendes Öl.

Schmp.: 66°C (Toluol), IR (KBr): 3400, 3325, 3213 ($NH_2$), 2219 (C=N), 1731 1709, 1694 $cm^{-1}$ (C=O)
$C_{11}H_{12}N_2O_4S$ (268,29)

| | | | | | |
|---|---|---|---|---|---|
| Ber.: | C 49,25 | H 4,51 | N 10,44 | O 23,85 | S 11,95 |
| Gef.: | C 49,4 | H 4,5 | N 10,4 | O 23,8 | S 11,8 |

Die Herstellung der Verbindung des Beispiels 8 erfolgt analog

**Beispiel 8**

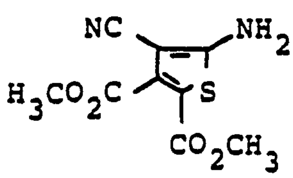

Schmp.: 153 °C (Toluol)

**Beispiel 9**

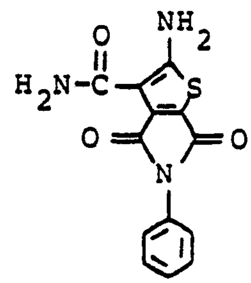

26,9 Teile 2-Amino-3-cyanthiophen-(4,5)-N-phenyldicarboximid werden in 100 Teilen konz. Schwefelsäure gelöst, die Mischung 0,5 h auf 40°C erwärmt und anschließend in 300 Teile Eiswasser gegeben. Der Niederschlag wird abgesaugt, mit Wasser gewaschen und getrocknet. Man erhält 19,4 Teile (68 % der Theorie) 2-Amino-3-carbamoyl-thiophen-(4,5)-N-phenyldicarboximid.

Schmp.: 294°C (n-Butanol), IR (KBr): 3396, 3365, 3250, 3172 ($NH_2$), 1744, 1703, 1683 $cm^{-1}$ (C=O)

$C_{13}H_9N_3O_3S$ (287,30)

| | | | | | |
|---|---|---|---|---|---|
| Ber.: | C 54,34 | H 3,16 | N 14,63 | O 16,71 | S 11,16 |
| Gef.: | C 54,3 | H 3,1 | N 14,3 | O 17,2 | S 11,1 |

**Beispiel 10**

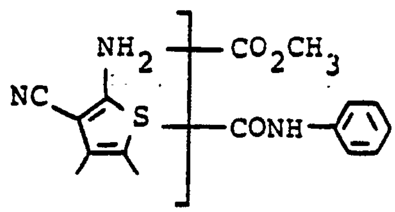

Eine Mischung aus 10,8 Teilen 2-Amino-3-cyan-thiophen-(4,5)-N-phenyldicarboximid, 4 Teilen Triethylamim und 80 Teilen Methanol wird 2 h zum Sieden erhitzt. Nach dem Abkühlen des Gemisches saugt man den entstandenen Niederschlag ab, wäscht ihn mit Methanol und trocknet ihn. Man erhält 7,6 Teile (63 % der Theorie) einer Verbindung der obigen Formel.

Schmp.: 273°C (Methylglykol), IR (KBr): 3380, 3300, 3195 (NH, $NH_2$), 2215 (C=N), 1694, 1633, 1616, 1608, 1596 $cm^{-1}$ (C=O)

$C_{14}H_{11}N_3O_3S$ (301,32)

| | | | | | |
|---|---|---|---|---|---|
| Ber.: | C 55,81 | H 3,68 | N 13,95 | O 15,93 | S 10,64 |
| Gef.: | C 55,7 | H 3,6 | N 13,9 | O 15,5 | S 10,9 |

**Beispiel 11**

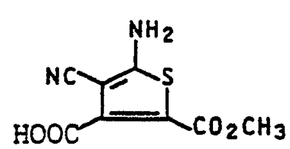

83,5 Teile Dichlormaleinsäureanhydrid werden unter Eiskühlung Portionsweise in 600 Teile einer 4,5-%-igen methanolischen Natriummethylatlösung eingetragen und die Mischung wird 1 h bei Raumtemperatur gerührt. Dann werden unter Eiskühlung 33 Teile Malonsäuredinitril und anschließend 180 Teile einer 30-%-igen Natriummethylat in Methanol zugegeben. Das Reaktionsgemisch wird 3 h bei Raumtemperatur gerührt. Unter erneuter Eiskühlung gibt man dann 50,5 Teile Triethylamin zu und leitet danach 17 Teile Schwefelwasserstoff gasförmig ein.

Die Mischung wird über Nacht gerührt, dann in 3000 Teile Wasser gegeben und unter Eiszugabe mit verd. Salzsäure angesäuert. Der entstandene Niederschlag wird abgesaugt, mit Wasser gewaschen und getrocknet. Man erhält 60,5 Teile (54 % der Theorie) 2-Amino-3-cyan-5-methoxycarbonylthiophen-4-carbonsäure.

Schmp.: 256° C (Eisessig), IR (KBr): 3314, 3164 ($NH_2$), 2220 (C≡N), 1710, 1655 $cm^{-1}$ (C≡O)
$C_8H_6N_2O_4S$ (226,21)

| | | | | | |
|---|---|---|---|---|---|
| Ber.: | C 42,48 | H 2,67 | N 12,38 | O 28,29 | S 14,17 |
| Gef.: | C 42,8 | H 2,9 | N 12,2 | O 28,6 | S 14,2 |

**Beispiel 12**

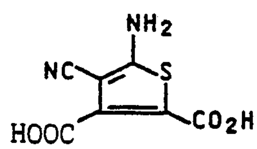

56,5 Teile Thiophencarbonsäure des Beispiels 11 werden in einer Lösung aus 200 Teilen Wasser und 20 Teilen Natriumhydroxid 0,5 h bei 50° C gerührt, dann filtriert man das Gemisch ab und säuert das Filtrat unter Eiszugabe mit verdünnter Salzsäure an. Der Niederschlag wird abgesaugt, mit Wasser gewaschen und getrocknet. Man erhält 51 Teile (96 % der Theorie) 2-Amino-3-cyan-thiophen-4,5-dicarbonsäure.

Schmp.: >300° C, IR (KBr): 3431, 3324, 3184 ($NH_2$), 3600-2300 (OH), 2220 (C≡N)
$C_7H_4N_2O_4S$ (212,19)

**Beispiel 13**

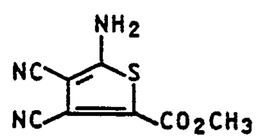

Man gibt unter Eiskühlung 15,3 Teile Phosphoroxytrichlorid in 80 Teile Dimethylformamid, rührt die Mischung noch 0,5 h bei 10° C und trägt dann 9 Teile Thiophencarbonsäure des Beispiels 11 ein. Die Lösung wird 2 h bei Raumtemperatur gerührt, dann in ein Gemisch aus 50 Teilen konz. wässriger Ammoniaklösung, 100 Teilen Eis und 100 Teilen Wasser gegeben. Der Niederschlag wird abgesaugt, mit Wasser gewaschen und getrocknet. Man erhält 9,7 Teile (87 % der Theorie) der Verbindung der Formel

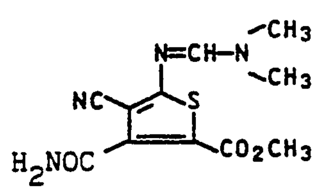

Schmp.: 224° C (Eisessig), IR (KBr): 3430, 3325 ($NH_2$), 2220 (C≡N), 1688, 1673, 1627 $cm^{-1}$ (C=O, C=N-)
$C_{11}H_{12}N_4O_3S$ (280,31)

| | | | | | |
|---|---|---|---|---|---|
| Ber.: | C 47,13 | H 4,32 | N 19,99 | O 17,12 | S 11,44 |
| Gef.: | C 47,3 | H 4,3 | N 19,3 | O 17,7 | S 11,5 |

53,7 Teile Phosphoroxytrichlorid werden unter Eiskühlung in 500 Teile Dimethylformamid eingetropft, anschließend rührt man das Gemisch noch 0,5 h bei 10°C. In die Lösung werden dann 98 Teile des gemäß Absatz 1 hergestellten Thiophencarbonsäureamids eingetragen, die Mischung wird 3 h bei Raumtemperatur gerührt- und dann auf 1500 Teile Eiswasser gefällt. Man erhält 76,3 Teile (83 % der Theorie) der Verbindung der Formel

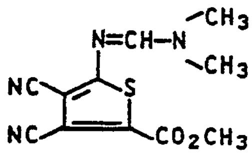

Schmp.: 201°C (Eisessig), IR (KBr): 2230, 2220 (C≡N), 1717 (C=O), 1630 $cm^{-1}$ (C=N-)
$C_{11}H_{10}N_4O_2S$ (262,29)

| | | | | | |
|---|---|---|---|---|---|
| Ber.: | C 50.37 | H 3,84 | N 21,36 | O 12,20 | S 12,22 |
| Gef.: | C 50,0 | H 3,9 | N 21,1 | O 12,7 | S 12,2 |

76 Teile davon werden in einem Gemisch aus 700 Teilen Ethanol und 70 Teilen konz. Salzsäure 3 h zum Sieden erhitzt. Wach dem Abkühlen auf Raumtemperatur wird der gebildete Niederschlag abgesaugt, zunächst mit Ethanol, dann mit Wasser gewaschen und getrocknet. Man erhält 61 Teile 2-Amino-3,4-dicyanthiophen-5-carbonsäuremethylester.
Schmp.: 282°C (Eisessig), IR (KBr): 3427, 3292, 3191 ($NH_2$), 2232, 2222 (C≡N), 1708 $cm^{-1}$ (C=O)
$C_8H_5N_3O_2S$ (207,21)

| | | | | | |
|---|---|---|---|---|---|
| Ber.: | C 46,37 | H 2,43 | N 20,28 | O 15,44 | S 15,47 |
| Gef.: | C 46,5 | H 2,5 | N 20,0 | O 16,1 | S 15,4 |

**Beispiel 14**

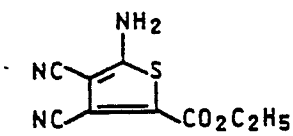

2-Amimo-3,4-dicyan-thiophen-5-carbonsäureethylester wird analog dem für Beispiel 13 beschriebenen Verfahren hergestellt.
Schmp.: 249°C (Eisessig), IR (KBr): 3321, 3190 ($NH_2$), 2219 (C≡N ),1685 (C=O)
$C_9H_7N_3O_2S$ (221,24)

| | | | | | |
|---|---|---|---|---|---|
| Ber.: | C 48,86 | H 3,19 | N 18,99 | O 14,46 | S 14,49 |
| Gef.: | C 48,7 | H 3,3 | N 18,5 | O 14,7 | S 14,9 |

**Patentansprüche**

1. Verbindungen der allgemeinen Formel I

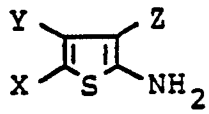
I,

in der
X und Y unabhängig voneinander Carboxyl, $C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxy)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, gegebenenfalls durch Chlor, Brom, Nitro oder Cyan substituiertes Phenoxycarbonyl, $C_1$- bis $C_4$-Alkylphenoxycarbonyl, Dimethylphenoxycarbonyl, Trimethylphenoxycarbonyl, Carbamoyl, $C_1$- bis $C_{12}$-AlkylcarbamoYl, Cyclohexylcarbamoyl, Allylcarbamoyl, 2-($C_1$- bis $C_4$-Alkoxy)-ethylcarbamoyl, 3-($C_1$- bis $C_4$-Alkoxy)-propylcar-

bamoyl, 2-Phenoxyethylcarbamoyl, 3-Phenoxypropylcarbamoyl, Phenylmethylcarbamoyl, 2-Phenylethylcarbamoyl, Phenylcarbamoyl, gegebenenfalls durch Chlor, Brom, Nitro oder Cyan substituiertes Phenylcarbamoyl, $C_1$- bis $C_{12}$-Alkylphenylcarbamoyl, Dimethylphenylcarbamoyl, Trimethylphenylcarbamoyl, $C_1$- bis $C_4$-Dialkylcarbamoyl, Methyl-cyclohexylcarbamoyl, Dicyclohexylcarbamoyl oder Reste der Formel

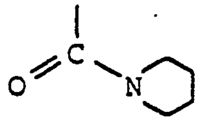 , 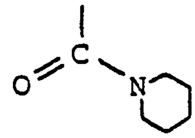 oder 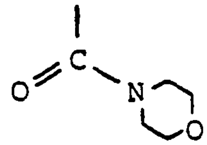 ,

X und Y zusammen ein Rest der Formel

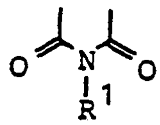 oder 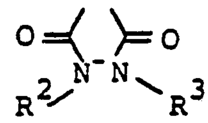 und

Z Cyan, Carboxyl, $C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxy)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, gegebenenfalls durch Chlor, Brom, Nitro oder Cyan substituiertes Phenoxycarbonyl, $C_1$- bis $C_4$-Alkylphenoxycarbonyl, Dimethylphenoxycarbonyl, Trimethylphenoxycarbonyl, Carbamoyl, $C_1$- bis $C_{12}$-Alkylcarbamoyl, Cyclohexylcarbamoyl, Allylcarbamoyl, 2-($C_1$- bis $C_4$-Alkoxy)-ethylcarbamoyl, 3-($C_1$- bis $C_4$-Alkoxy)-propylcarbamoyl, 2-Phenoxyethylcarbamoyl, 3-Phenoxypropylcarbamoyl, Phenylmethylcarbamoyl, 2-Phenylethylcarbamoyl, Phenylcarbamoyl, gegebenenfalls durch Chlor, Brom, Nitro oder Cyan substituiertes Phenylcarbamoyl, $C_1$- bis $C_{12}$-Alkylphenylcarbamoyl, Dimethylphenylcarbamoyl, Trimethylphenylcarbamoyl, $C_1$- bis $C_4$-Dialkylcarbamoyl, Methyl-cyclohexylcarbamoyl, Dicyclohexylcarbamoyl oder Reste der Formel

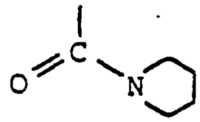 , 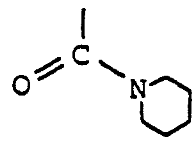 oder 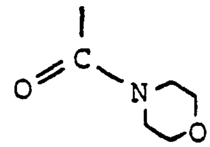 ,

sind, wobei

$R^1$ Wasserstoff, $C_1$- bis $C_{12}$-Alkyl, Cyclohexyl, Allyl, 2-($C_1$- bis $C_4$-Alkoxy)-ethyl, 3-($C_1$- bis $C_4$-Alkoxy)-propyl, 2-Phenoxyethyl, 3-Phenoxypropyl, Phenylmethyl, Phenylethyl oder gegebenenfalls durch Chlor, Brom, Cyan oder Nitro substituiertes Phenyl, $C_1$- bis $C_{12}$-Alkylphenyl, Dimethylphenyl oder Trimethylphenyl und

$R^2$ und $R^3$ Wasserstoff oder $C_1$- bis $C_{12}$-Alkyl oder einer der Reste $R^2$ und $R^3$ auch gegebenenfalls durch Chlor, Brom, Cyan oder Nitro substituiertes Phenyl sind.

2. Verbindungen gemäß Anspruch 1 der Formel

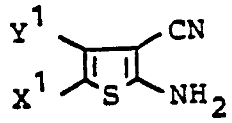 I a,

in der

$X^1$ und $Y^1$ unabhängig voneinander Carboxyl, $C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxy)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, gegebenenfalls durch Chlor, Brom, Nitro oder Cyan substituiertes Phenoxycarbonyl, $C_1$- bis $C_4$-Alkylphenoxycarbonyl, Dimethylphenoxycarbonyl oder Trimethylphenoxycarbonyl oder

$X^1$ und $Y^1$ zusammen ein Rest der Formel

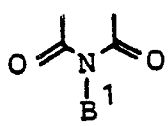

sind,

wobei

$B^1$ Wasserstoff, $C_1$- bis $C_{12}$-Alkyl Cyclohexyl, Phenylmethyl, Phenylethyl, Phenyl $C_1$- bis $C_{12}$-Alkylphenyl, Dimethylphenyl, Trimethylphenyl oder durch Chlor, Brom, Cyan oder Nitro substituiertes Phenyl ist.

3. Verbindungen gemäß Anspruch 2, bei denen $X^1$ und $Y^1$ unabhängig voneinander $CONHB^1$ sind und wobei $B^1$ die angegebene Bedeutung hat.

4. Verbindungen gemäß Anspruch 1 der Formel

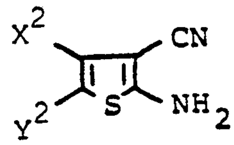

in der einer der beiden Reste $X^2$ und $Y^2$ $CONHB^1$ und der andere Di-($C_1$- bis $C_{12}$-Alkyl)-carbamoyl, Carboxyl, $C_1$- bis $C_{12}$-Alkoxycarbonyl, Phenoxycarbonyl oder ein Rest der Formel

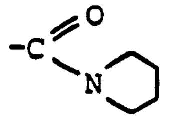 , 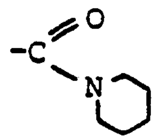 oder 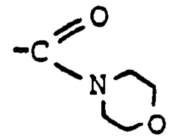

sind und
$B^1$ die angegebene Bedeutung hat.

5. Verbindungen gemäß Anspruch 1 der Formel

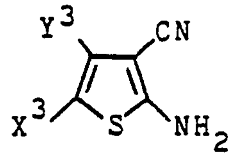

in der
$X^3$ $C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxy)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, gegebenenfalls durch Chlor, Brom, Nitro oder Cyan substituiertes Phenoxycarbonyl, $C_1$- bis $C_4$-Alkylphenoxycarbonyl, Dimethylphenoxycarbonyl oder Trimethylphenoxycarbonyl und
$Y^3$ Carboxyl, Cyan, Carbamoyl, $C_1$- bis $C_{12}$-Alkylcarbamoyl, Cyclohexylcarbamoyl, Allylcarbamoyl, 2-($C_1$- bis $C_4$-Alkoxy)-ethylcarbamoyl, 3-($C_1$- bis $C_4$-Alkoxy)-propylcarbamoyl, 2-Phenoxyethylcarbamoyl, 3-Phenoxypropylcarbamoyl, Phenylmethylcarbamoyl, 2-Phenylethylcarbamoyl, Phenylcarbamoyl, gegebenenfalls durch Chlor, Brom, Nitro oder Cyan substituiertes Phenylcarbamoyl, $C_1$- bis $C_{12}$-Alkylphenylcarbamoyl, Dimethylphenylcarbamoyl, Trimethylphenylcarbamoyl, $C_1$- bis $C_4$-Dialkylcarbamoyl, Methyl-cyclohexylcarbamoyl, Dicyclohexylcarbamoyl oder Reste der Formel

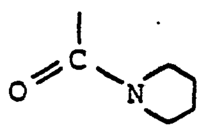 , 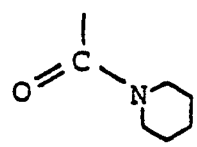 oder 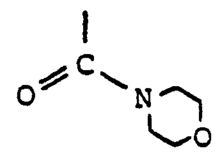 ,

$C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxy)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, gegebenenfalls durch Chlor, Brom, Nitro oder Cyan substituiertes Phenoxycarbonyl, $C_1$- bis $C_4$-Alkylphenoxycarbonyl, Dimethylphenoxycarbonyl oder Trimethylphenoxycarbonyl sind.

## Claims

1. A compound of the formula I

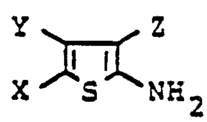 I

where
X and Y independently of one another are each carboxyl, $C_1$-$C_{12}$-alkoxycarbonyl, cyclohexyloxycarbonyl, allyloxycarbonyl, 2-($C_1$-$C_4$-alkoxy)-ethoxycarbonyl, 3-($C_1$-$C_4$-alkoxy)-propoxycarbonyl, 2-phenoxyethoxycarbonyl, 3-phenoxypropoxycarbonyl, phenylmethoxycarbonyl, 2-phenylethoxycarbonyl, phenoxycarbonyl, phenoxycarbonyl which is substituted by chlorine, bromine, nitro or cyano, or $C_1$-$C_4$-alkylphenoxycarbonyl, dimethylphenoxycarbonyl, trimethylphenoxycarbonyl, carbamyl, $C_1$-$C_{12}$-alkylcarbamyl, cyclohexylcarbamyl,

allylcarbamyl, 2-($C_1$-$C_4$-alkoxy)-ethylcarbamyl, 3-($C_1$-$C_4$-alkoxy)-propylcarbamyl, 2-phenoxy-ethylcarbamyl, 3-phenoxypropylcarbamyl, phenylmethylcarbamyl, 2-phenylethylcarbamyl, phenylcarbamyl, phenylcarbamyl which is substituted by chlorine, bromine, nitro or cyano, or $C_1$-$C_{12}$-alkylphenylcarbamyl, dimethylphenylcarbamyl, trimethylphenylcarbamyl, $C_1$-$C_4$-dialkylcarbamyl, methylcyclohexylcarbamyl, dicyclohexylcarbamyl or a radical of the formula

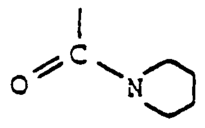 , 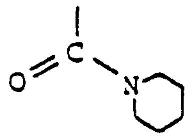 or 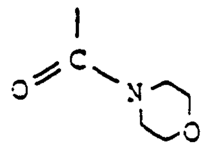

X and Y together form a radical of the formula

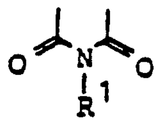 or 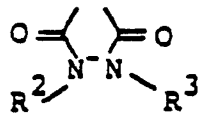

and

Z is cyano, carboxyl, $C_1$-$C_{12}$-alkoxycarbonyl, cyclohexyloxycarbonyl, allyloxycarbonyl, 2-($C_1$-$C_4$-alkoxy)-ethoxycarbonyl, 3-($C_1$-$C_4$-alkoxy)-propoxycarbonyl, 2-phenoxyethoxycarbonyl, 3-phenoxypropoxycarbonyl, phenylmethoxycarbonyl, 2-phenylethoxycarbonyl, phenoxycarbonyl, phenoxycarbonyl which is substituted by chlorine, bromine, nitro or cyano, or $C_1$-$C_4$-alkylphenoxycarbonyl, dimethylphenoxycarbonyl, trimethylphenoxycarbonyl, carbamyl, $C_1$-$C_{12}$-alkylcarbamyl, cyclohexylcarbamyl, allylcarbamyl, 2-($C_1$-$C_4$-alkoxy)-ethylcarbamyl, 3-($C_1$-$C_4$-alkoxy)-propylcarbamyl, 2-phenoxyethylcarbamyl, 3-phenoxypropylcarbamyl, phenylmethylcarbamyl, 2-phenylethylcarbamyl, phenylcarbamyl, phenylcarbamyl which is substituted by chlorine, bromine, nitro or cyano, or $C_1$-$C_{12}$ alkylphenylcarbamyl, dimethylphenylcarbamyl, trimethylphenylcarbamyl, $C_1$-$C_4$-dialkylcarbamyl, methylcyclohexylcarbamyl, dicyclohexylcarbamyl or a radical of the formula

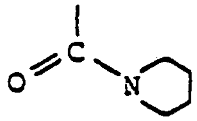 , 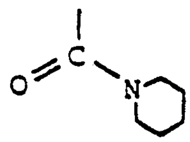 or 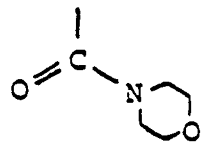 ,

and

$R^1$ is hydrogen, $C_1$-$C_{12}$-alkyl, cyclohexyl, allyl, 2-($C_1$-$C_4$-alkoxy)-ethyl, 3-($C_1$-$C_4$-alkoxy)-propyl, 2-phenoxyethyl, 3-phenoxypropyl, phenylmethyl, phenylethyl or phenyl which may be substituted by chlorine, bromine, cyano or nitro, or $C_1$-$C_{12}$-alkylphenyl, dimethylphenyl or trimethylphenyl and

$R^2$ and $R^3$ are each hydrogen or $C_1$-$C_{12}$-alkyl, or one of the radicals $R^2$ and $R^3$ may furthermore be phenyl which may be substituted by chlorine, bromine, cyano or nitro.

2. A compound as claimed in claim 1 of the formula

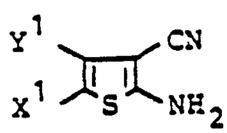 I a

where

$X^1$ and $Y^1$ independently of one another are each carboxyl, $C_1$-$C_{12}$-alkoxycarbonyl, cyclohexyloxycarbonyl, allyloxycarbonyl, 2-($C_1$-$C_4$-alkoxy)-ethoxycarbonyl, 3-($C_1$-$C_4$-alkoxy)-propoxycarbonyl, 2-phenoxyethoxycarbonyl, 3-phenoxypropoxycarbonyl, phenylmethoxycarbonyl, 2-phenylethoxycarbonyl, phenoxycarbonyl, phenoxycarbonyl which is substituted by chlorine, bromine, nitro or cyano, or $C_1$-$C_4$-alkylphenoxycarbonyl, dimethylphenoxycarbonyl or trimethylphenoxycarbonyl, or $X^1$ and $Y^1$ together form a radical of the formula

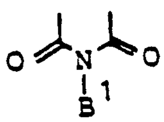

where

$B^1$ is hydrogen, $C_1$-$C_{12}$-alkyl, cyclohexyl, phenylmethyl, phenylethyl, phenyl, $C_1$-$C_{12}$-alkylphenyl, dimethylphenyl, trimethylphenyl or phenyl which is substituted by chlorine, bromine, cyano or nitro.

3. A compound as claimed in claim 2 in which $X^1$ and $Y^1$ independently of one another are each $CONHB^1$ and $B^1$ has the stated meanings.

4. A compound as claimed in claim 1 of the formula

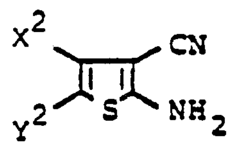

where one of the two radicals $X^2$ and $Y^2$ is $CONHB^1$ and the other is di-($C_1$-$C_{12}$alkyl)-carbamyl, carboxyl, $C_1$-$C_{12}$-alkoxycarbonyl, phenoxycarbonyl or a radical of the formula

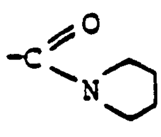 , 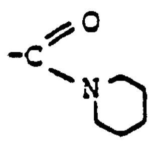 or 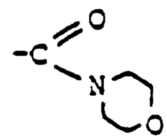

and $B^1$ has the stated meanings.

5. A compound as claimed in claim 1 of the formula

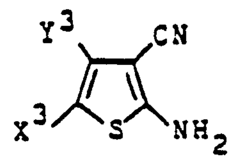

where

$X^3$ is $C_1$-$C_{12}$-alkoxycarbonyl, cyclohexyloxycarbonyl, allyloxycarbonyl, 2-($C_1$-$C_4$-alkoxy)-ethoxycarbonyl, 3-($C_1$-$C_4$-alkoxy)-propoxycarbonyl, 2-phenoxyethoxycarbonyl, 3-phenoxypropoxycarbonyl, phenylmethoxycarbonyl, 2-phenylethoxycarbonyl, phenoxycarbonyl, phenoxycarbonyl which is substituted by chlorine, bromine, nitro or cyano, or $C_1$-$C_4$-alkylphenoxycarbonyl, dimethylphenoxycarbonyl or trimethylphenoxycarbonyl and

$Y^3$ is carboxyl, cyano, carbamyl, $C_1$-$C_{12}$-alkylcarbamyl, cyclohexylcarbamyl, allylcarbamyl, 2-($C_1$-$C_4$-alkoxy)-ethylcarbamyl, 3-($C_1$-$C_4$-alkoxy)-propylcarbamyl, 2-phenoxyethylcarbamyl, 3-phenoxypropylcarbamyl, phenylmethylcarbamyl, 2-phenylethylcarbamyl, phenylcarbamyl, phenylcarbamyl which is substituted by chlorine, bromine, nitro or cyano, or $C_1$-$C_{12}$-alkylphenylcarbamyl, dimethylphenylcarbamyl, trimethylphenylcarbamyl, $C_1$-$C_4$-dialkylcarbamyl, methylcyclohexylcarbamyl, dicyclohexylcarbamyl or a radical of the formula

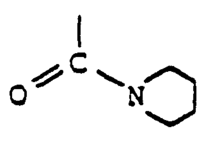 , 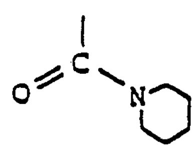 or 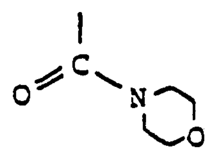 ,

$C_1$-$C_{12}$-alkoxycarbonyl, cyclohexyloxycarbonyl, allyloxycarbonyl, 2-($C_1$-$C_4$-alkoxy)-ethoxycarbonyl, 3-($C_1$-$C_4$-alkoxy)-propoxycarbonyl, 2-phenoxyethoxycarbonyl, 3-phenoxypropoxycarbonyl, phenylmethoxycarbonyl, 2-phenylethoxycarbonyl, phenoxycarbonyl, phenoxycarbonyl which is substituted by chlorine, bromine, nitro or cyano, or $C_1$-$C_4$-alkylphenoxycarbonyl, dimethylphenoxycarbonyl or trimethylphenoxycarbonyl.

**Revendications**

1. Composés de formule générale I

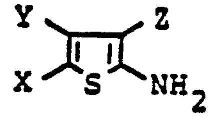

I

dans laquelle

X et Y représentent indépendamment l'un de l'autre des groupements carboxyle, (alcoxy en $C_1$-$C_{12}$)-carbonyle, cyclohexyloxycarbonyle, allyloxycarbonyle, 2-(alcoxy en $C_1$-$C_4$)-éthoxycarbonyle, 3-(alcoxy en $C_1$-$C_4$)-propyloxycarbonyle, 2-phénoxyéthoxycarbonyle, 3-phénoxypropyloxycarbonyle, phénylméthoxycarbonyle, 2-phényléthoxycarbonyle, phénoxycarbonyle, phénoxycarbonyle éventuellement substitué par des radicaux chloro, bromo, nitro ou cyano, (alkyl en $C_1$-$C_4$)-phénoxycarbonyle, diméthylphénoxycarbonyle, triméthylphénoxycarbonyle, carbamoyle, (alkyl en $C_1$-$C_{12}$)-carbamoyle, cyclohexylcarbamoyle, allylcarbamoyle, 2-(alcoxy en $C_1$-$C_4$)-éthylcarbamoyle, 3-(alcoxy en $C_1$-$C_4$)-propylcarbamoyle, 2-phénoxyéthylcarbamoyle, 3-phénoxypropylcarbamoyle, phénylméthylcarbamoyle, 2-phényléthylcarbamoyle, phénylcarbamoyle, phénylcarbamoyle éventuellement substitué par des radicaux chloro, bromo, nitro ou cyano, (alkyl en $C_1$-$C_{12}$)-phénylcarbamoyle,

diméthylphénylcarbamoyle, triméthylphénylcarbamoyle, di(alkyl en $C_1$-$C_4$)-carbamoyle, méthylcyclohexylcarbamoyle, dicyclohexylcarbamoyle ou des restes de formule

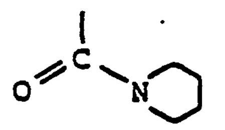 , 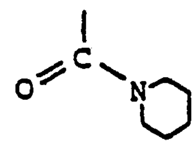 ou 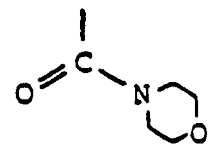

X et Y représentent ensemble un reste de formule

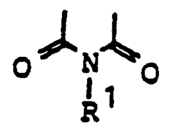 ou 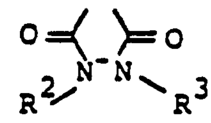

Z est un groupement cyano, carboxyle, (alcoxy en $C_1$-$C_{12}$)-carbonyle, cyclohexyloxycarbonyle, allyloxycarbonyle, 2-(alcoxy en $C_1$-$C_4$)-éthoxycarbonyle, 3-(alcoxy en $C_1$-$C_4$)-propyloxycarbonyle, 2-phénoxyéthoxycarbonyle, 3-phénoxypropyloxycarbonyle, phénylméthoxycarbonyle, 2-phenyléthoxycarbonyle, phénoxycarbonyle, phénoxycarbonyle éventuellement substitué par des radicaux chloro, bromo, nitro ou cyano, (alkyl en $C_1$-$C_4$)-phénoxycarbonyle, diméthylphénoxycarbonyle, triméthylphénoxycarbonyle, carbamoyle, (alkyl en $C_1$-$C_{12}$)-carbamoyle, cyclohexylcarbamoyle, allylcarbamoyle, 2-(alcoxy en $C_1$-$C_4$)-éthylcarbamoyle, 3-(alcoxy en $C_1$-$C_4$)-propylcarbamoyle, 2-phénoxyéthylcarbamoyle, 3-phénoxypropylcarbamoyle, phénylméthylcarbamoyle, 2-phényléthylcarbamoyle, phénylcarbamoyle, phénylcarbamoyle éventuellement substitué par des radicaux chloro, bromo, nitro ou cyano, (alkyl en $C_1$-$C_{12}$)-phénylcarbamoyle, diméthylphénylcarbamoyle, triméthylphénylcarbamoyle, di(alkyl en $C_1$-$C_4$)-carbamoyle, méthylcyclohexylcarbamoyle, dicyclohexylcarbamoyle ou un reste de formule

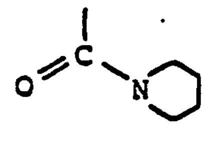 , 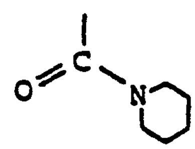 ou 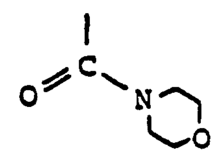

dans lesquelles

$R^1$ est un atome d'hydrogène ou un groupement alkyle en $C_1$-$C_{12}$, cyclohexyle, allyle, 2-(alcoxy en $C_1$-$C_4$)-éthyle, 3-(alcoxy en $C_1$-$C_4$)-propyle, 2-phénoxyéthyle, 3-phénoxypropyle, phénylméthyle, phényléthyle ou phényle éventuellement substitué par des radicaux chloro, bromo, cyano ou nitro, (alkyl en $C_1$-$C_{12}$)-phényle, diméthylphényle ou triméthylphényle, et

$R^2$ et $R^3$ sont des atomes d'hydrogène ou des groupements alkyle en $C_1$-$C_{12}$ ou l'un des restes $R^2$ et $R^3$ est aussi un reste phényle éventuellement substitué par des radicaux chloro, bromo, cyano ou nitro.

2. Composés selon la revendication 1, de formule

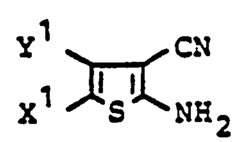 I a,

dans laquelle

$X^1$ et $Y^1$ représentent indépendamment l'un de l'autre des groupements carboxyle, (alcoxy en $C_1$-$C_{12}$)-carbonyle, cyclohexyloxycarbonyle, allyloxycarbonyle, 2-(alcoxy en $C_1$-$C_4$)-éthoxycarbonyle, 3-(alcoxy en $C_1$-$C_4$)-propyloxycarbonyle, 2-phénoxyethoxycarbonyle, 3-phénoxypropyloxycarbonyle, phénylméthoxycarbonyle, 2-phényléthoxyoarbonyle, phénoxycarbonyle, phénoxycarbonyle éventuellement substitué par des radicaux chloro, bromo, nitro ou cyano, (alkyl en $C_1$-$C_4$)-phénoxycarbonyle, dimethylphénoxycarbonyle ou triméthylphénoxycarbonyle ou $X^1$ et $Y^1$ représentent ensemble un reste de formule

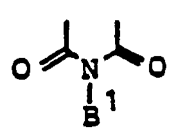

dans laquelle

$B^1$ est un atome d'hydrogène ou un groupement alkyle en $C_1$-$C_{12}$, cyclohexyle, phénylméthyle, phényléthyle, phényle, (alkyl en $C_1$-$C_{12}$)-phényle, dimethylphényle, triméthylphényle ou phényle substitué par des radicaux chloro, bromo, cyano ou nitro.

3. Composés selon la revendication 2, dans laquelle $X^1$ et Y' sont indépendamment l'un de l'autre des restes $CONHB^1$ où $B^1$ a la signification donnée.

4. Composés selon la revendication 1, de formule

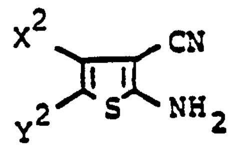

dans laquelle l'un des deux restes $X^2$ et $Y^2$ est $CONHB^1$ et l'autre est un groupement di(alkyl en $C_1$-$C_{12}$)-carbamoyle, carboxyle, (alcoxy en $C_1$-$C_{12}$)-carbonyle, phénoxycarbonyle ou un reste de formule

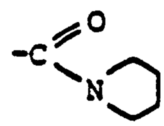 , 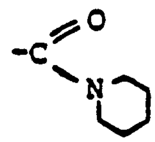 ou 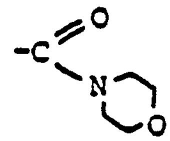

et $B^1$ a la signification donnée.

5. Composés selon la revendication 1 de formule

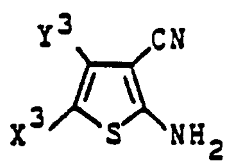

dans laquelle

$X^3$ est un groupement (alcoxy en $C_1$-$C_{12}$)-carbonyle, cyclohexyloxycarbonyle, allyloxycarbonyle, 2-(alcoxy en $C_1$-$C_4$)-éthoxycarbonyle, 3-(alcoxy en $C_1$-$C_4$)-propyloxy-carbonyle, 2-phénoxyéthoxycarbonyle, 3-phénoxypropyloxycarbonyle, phénylmethoxycarbonyle, 2-phényléthoxycarbonyle, phénoxycarbonyle, phénoxycarbonyle éventuellement susbtitué par des radicaux chloro, bromo, nitro ou cyano, (alkyl en $C_1$-$C_4$)-phénoxycarbonyle, diméthylphénoxycarbonyle ou triméthylphénoxycarbonyle et

$Y^3$ est un groupement carboxyle, cyano, carbamoyle, (alkyl en $C_1$-$C_{12}$)-carbamoyle, cyclohexylcarbamoyle, allylcarbamoyle, 2-(alcoxy en $C_1$-$C_{12}$)-éthylcarbamoyle, 3-(alcoxy en $C_1$-$C_4$)-propylcarbamoyle, 2-phénoxyéthylcarbamoyle, 3-phénoxypropylcarbamoyle, phénylméthylcarbamoyle, 2-phényléthylcarbamoyle, phénylcarbamoyle, phénylcarbamoyle éventuellement substitué par des radicaux chloro, bromo, nitro ou cyano, (alkyl en $C_1$-$C_{12}$)-phénylcarbamoyle, diméthylphénylcarbamoyle, triméthylphénylcarbamoyle, di(alkyl en $C_1$-$C_4$)-carbamoyle, méthylcyclohexylcarbamoyle, dicyclohexylcarbamoyle ou un reste de formule

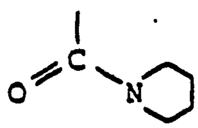 , 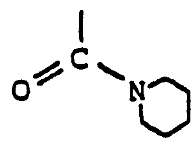 ou 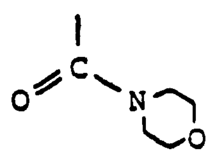

(alcoxy en $C_1$-$C_{12}$)-carbonyle, cyclohexyloxycarbonyle, allyloxycarbonyle, 2-(alcoxy en $C_1$-$C_4$)-éthoxycarbonyle, 3-(alcoxy en $C_1$-$C_4$)-propyloxycarbonyle, 2-phénoxyéthoxycarbonyle, 3-phénoxypropyloxycarbonyle, phénylméthoxycarbonyle, 2-phényléthoxycarbonyle, phénoxycarbonyle, phénoxycarbonyle éventuellement substitué par des radicaux chloro, bromo, nitro ou cyano, (alkyl en $C_1$-$C_4$)-phénoxycarbonyle, dimethylphénoxycarbonyle ou triméthylphénoxycarbonyle.